**Europäisches Patentamt**

**European Patent Office**

**Office européen des brevets**

(19)

(11) Veröffentlichungsnummer: **0 182 078**
**B1**

(12) # EUROPÄISCHE PATENTSCHRIFT

(45) Veröffentlichungstag der Patentschrift: **23.01.91**

(21) Anmeldenummer: **85112927.0**

(22) Anmeldetag: **11.10.85**

(51) Int. Cl.⁵: **B 01 J 27/18**, B 01 J 23/22,
B 01 J 35/10, C 07 C 51/215,
C 07 C 51/25, C 07 C 57/04

(54) **Katalysator für Oxidationsreaktionen und Verfahren zu dessen Herstellung.**

(30) Priorität: **20.11.84 IT 2367184**

(43) Veröffentlichungstag der Anmeldung:
**28.05.86 Patentblatt 86/22**

(45) Bekanntmachung des Hinweises auf die
Patenterteilung:
**23.01.91 Patentblatt 91/04**

(84) Benannte Vertragsstaaten:
**AT BE CH DE FR GB IT LI LU NL SE**

(56) Entgegenhaltungen:
**GB-A-2 118 060**
**US-A-4 092 269**
**US-A-4 187 235**
**US-A-4 435 521**

(73) Patentinhaber: **ALUSUISSE ITALIA S.p.A.**
**Via Vittor Pisani, 31**
**I-20124 Milano (IT)**

(72) Erfinder: **Fumagalli, Carlo, Dr.**
**Via A. Moro 4**
**Valbrembo Prov. Bergamo (IT)**
Erfinder: **Stefani, Giancarlo, Dr.**
**Viale Zavaritt 193**
**Gorle (Prov. Bergamo) (IT)**

(74) Vertreter: **von Füner, Alexander, Dr. et al**
**Patentanwälte v. Füner, Ebbinghaus, Finck**
**Mariahilfplatz 2 & 3**
**D-8000 München 90 (DE)**

EP 0 182 078 B1

Courier Press, Leamington Spa, England.

# EP 0 182 078 B1

**Beschreibung**

Die Erfindung betrifft einen Katalysator für die partielle Oxidation von Kohlenwasserstoffen zu Maleinsäureanhydrid, enthaltend ein Vanadium-Phosphor-Oxidgemisch, ein Verfahren zur Herstellung dieses Katalysators und seine Verwendung.

Es ist bekannt, V—P—O-Komplexoxide als Katalysatoren für die Herstellung von Maleinsäureanhydrid aus n-Butan einzusetzen. Aus der US—PS 3 293 268 ist ein Verfahren zur Herstellung von V—P—O-Komplexen aus einer wässrigen Lösung von Salzsäure bekannt, mit $V_2O_5$, $H_3PO_4$ und Oxalsäure als Ausgangsmaterialien. Der V—P—O-Komplex wird durch Eindampfen des Reaktionsgemisches bis zur Trockene gewonnen. Nach dem Formen und Calcinieren des V—P—O-Komplexes werden molare Ausbeuten von nur 35—40% bei Temperaturen von 550—575°C erhalten.

Eine Verbesserung des Verfahrens nach US—PS 3 293 268 wird in der US—PS 4 100 106 beschrieben. Gemäss diesem Verfahren wird ein V—P—O-Komplex aus konzentrierter Salzsäure durch Wasserzugabe ausgefällt. Dadurch erzielt man ein kristallines Produkt, charakterisiert durch Röntgenspektrogramme, und nach entsprechender Konditionierung werden beim Einsatz des Katalysators für die Oxidation von Butan zu Maleinsäureanhydrid Ausbeuten von rund 55% bei Reaktionstemperaturen von 400—420°C erreicht. Diesem Verfahren haftet der Nachteil an, auf die hochkorrosive Salzsäure angewiesen zu sein.

Die Reduktion von $V_2O_5$ mit organischen Verbindungen ist seit einiger Zeit bekannt: J. S. Litter, W. A. Waters; J. Chem. Soc. *1959*, Seiten 1299—1305.

Ebenso ist die Reduktion von $V_2O_5$ in alkoholischer Lösung, enthaltend HCl bekannt aus: Koppel et al, Z. Anorg. Chem. *45*, [1905], Seiten 346—351.

Die US—PS 3 864 280 beschreibt die Herstellung eines V—P—O-Komplexes in Gegenwart von gasförmigem HCl. Sobald die Reduktion von $V_2O_5$ beendet ist, wird $H_3PO_4$ zugesetzt und der gebildete V—P—O-Komplex wird durch Verdampfen des organischen Lösungsmittels isoliert. Der V—P—O-Komplex wird durch ein streng einzuhaltendes Verfahren bei Temperaturen bis zu 450°C konditioniert und dabei in eine kristalline Phase, genannt "B-Phase" umgewandelt, die den aktiven Katalysator darstellt. Die Konditionierung dieses Katalysators ist für die Katalysatorleistung von grösster Wichtigkeit und bedarf eines sehr spezifischen Verfahrens, das genau eingehalten werden muss (Temperaturerhöhung von 3°C/min.), was sich bei industriellen Massstäben schlecht realisieren lässt. Weiters wird die geeignete spezifische Oberfläche mit 7 bis 50 m2/g angegeben und es wird beschrieben, dass die Aktivität des Katalysators mit zunehmender Oberfläche steigt.

Die DE—OS 3 010 710 nennt im Verfahren zur Herstellung eines V—P—O-Komplexes in einem organischen Lösungsmittel: Die $V_2O_5$-Reduktion zum Vanadium (IV) wird in der Gegenwart von $H_3PO_3$ und $H_3PO_4$ ausgeführt. Alle Reagentien werden zu Beginn der Reaktion zusammengegeben. Der so erhaltene Katalysator verlangt nach einer langen Aktivierungszeit, die besten Resultate werden mit entsprechendem Verlust an Produktionskapazität erst nach 10—20 Tagen erreicht.

Die GB-Anm. 2 118 060 beschreibt die Herstellung von Katalysatoren aus V—P—O-Komplexoxiden durch Mischen eines kristallinen-V—P—O-Komplexoxides mit einer wässrigen Lösung, welche Vanadium und Phosphor enthält, une einem Kieselsäuresol, Sprühtrocknen der so erhaltenen Suspension und Kalzinieren der so erhaltenen festen Partikel. Das kristalline V—P—O-Komplexoxid wird in wässriger Lösung aus $V_2O_5$ und Phosphorsäure mit Oxalsäure, Hydrazin, Hydroxylamin, Milchsäure oder phosphoriger Säure als Reduktionsmittel hergestellt. Der fertige Katalysator weist eine charakteristische Porenverteilung auf, die für die katalytischen Eigenschaften wesentlich zu sein scheint. In der US—PS 4 435 521 wird die Herstellung von V—P—O-Katalysatoren durch partielle Reduktion einer Verbindung des fünfwertigen Vanadiums, insbesondere $V_2O_5$, mit primären oder sekundären Alkoholen, insbesondere Isobutanol, und anschliessende Zugabe von Phosphorsäure beschrieben. Die Reaktion wird in siedender Lösung durchgeführt, wobei das gebildete Wasser durch azeotrope Destillation entfernt wird.

In allen bisher bekannt gewordenen Verfahren zur Herstellung von V—P—O-Komplexoxiden in organischen Lösungsmitteln war die Vanadium-(V)-Komponente von Beginn der Reaktion an im Reaktionsgemisch. In einigen Fällen wird die Phosphorkomponente am Ende der Reduktion des Vanadiums (V) zum Vanadium (IV) zugegeben, in anderen Fällen ist auch die Phosphorkomponente von Beginn an im Reaktionsgemisch enthalten.

Nach der Aufgabe vorliegender Erfindung wird ein Katalysator und ein Verfahren zu dessen Herstellung beschrieben, der sich gegenüber bekannten Katalysatoren durch eine leichtere und schnellere Konditionierung, und insbesondere durch höhere Aktivität, Selektivität und Produktivität auszeichnet.

Die gestellte Aufgabe wird erfindungsgemäß gelöst durch einen Katalysator für die Oxidation von Kohlenwasserstoffen zu Maleinsäureanhydrid, enthaltend ein Vanadium-Phosphor-Oxidgemisch und gegebenenfalls aktivitätserhöhende Zusätze, wobei der Katalysator pro Atom Vanadium 1 bis 1,3 Atome Phosphor enthält, der dadurch gekennzeichnet ist, daß der Katalysator derart hergestellt wird, daß in einem oganischen Lösungsmittel die phosphorhaltige Verbindung vorgelegt wird und innerhalb von 0,5 bis 4 Stunden, vorzugsweise 0,8 bis 2 Stunden, kontinuierlich die Vanadiumhaltige Verbindung zugegeben wird, wobei pro Atom Vanadium 1 bis 1,3 Atome Phosphor eingesetzt werden und das während der Reaktion gebildete Wasser laufend unmittelbar aus dem reaktionsgemisch entfernt wird, anschließend das Reaktionsgemisch getrennt und der V—P—O-Komplex als Feststoff isoliert und dieser bei Temperaturen von 90 bis 150°C getrocknet und bei Temperaturen von 200 bis 300°C zum Katalysator aktiviert wird.

2

Vorzugsweise ist der Katalysator so ausgebildet, daß wenigstens 90% der Katalysatorpartikel vor der Konfektionierung einen Durchmesser zwischen 1 und 5 µm aufweisen.

Der erfindungsgemässe Katalysator weist ein Porenvolumen der Poren mit einem Radius von 100 bis 1'000 Å von mindestens 30% des totalen Porenvolumens der Poren mit einem Radius von kleiner als 10'000 Å auf.

Vorzugsweise beträgt das Porenvolumen der Poren mit einem Radius von 100 bis 1'000 Å mindestens 40% des totalen Porenvolumens der Poren mit einem Radius von kleiner als 10'000 Å.

Idealerweise strebt der Wert für das porenvolumen der Poren mit einem Radius von 100 bis 1'000 Å gegen 100% des totalen Porenvolumens der Poren mit einem Radius von kleiner als 10'000 Å.

Eine vergleichbare Porenverteilung weist der in der GB-Anm. 2 118 060 beschriebene Katalysator auf.

Zur Definition des Porenvolumens werden nur Poren mit einem Radius von weniger als 10'000 Å in Betracht gezogen. Grössere Poren haben keine Bedeutung mehr, da sie schon im Bereich des Durchmesser der einzelnen Partikel liegen. Bekanntlich entspricht 1 µm jeweils 10'000 Å.

Die Porenvolumen werden zweckmässig nach dem der Fachwelt bekannten Verfahren der Quecksilberporosimetrie bestimmt.

Der erfindungsgemässe Katalysator liegt nach der Herstellung und vor einer Konfektionierung in feinster Pulverform vor. Nach der Erfindung weisen die Partikel des Pulvers zweckmässig zu 90% einen Durchmesser zwischen 1 bis 5 µm und durchscnittlich 2 bis 4 µm auf.

Der erfindungsgemässe Katalysator zeigt eine hohe Selektivität, eine hohe Aktivität und eine gleichmässig hohe Leistungsfähigkeit. So ist es möglich, den Katalysator über längste Zeiträume ohne Leistungseinbusse unter hohen spezifischen Speisungsraten, die beispielsweise bis zu 150 g n-Butan pro Stunde pro Kilogramm Katalysator betragen können, und mit hohen Ausbeuten von nahezu 100 kg Maleinsäureanhydrid pro 100 kg zugeführtem Butan, was einer stündlichen Maleinsäureanhydridproduktion von 150 g/kg Katalysator entspricht, zu betrieben.

Wird der Katalysator mit einer niedrigeren spezifischen Speisungsrate für die Umsetzung von Kohlenwasserstoffen, z.B. n-Butan, betrieben, ist es möglich, mengenmässige Ausbeuten um 110% zu erhalten.

In einer zweckmässigen Ausführungsform enthält der Katalysator pro Atom Vanadium 1,05 bis 1,2 Atome Phosphor.

Im weiteren kann der Katalysator noch einen oder mehrere aktivitätserhöhende Zusätze (Promotoren) aus der Reihe von Li, Ti, Zr, Hf, Cr, Mo, Mn, Fe, Co, Ni, Cu, Zn, B, Si, Sn oder Bi enthalten. Solche Zusätze sind an sich bekannt, ihr Einsatz ist beispielsweise in der US—PS 4 435 521 beschrieben.

Das erfindungsgemässe Verfahren ist dadurch gekennzeichnet, dass in einem organischen Lösungsmittel eine phosphorhaltige Verbindung vorgelegt und innerhalb 0,5 bis 4 Stunden, vorzugsweise 0,8 bis 2 Stunden, kontinuierlich eine vanadiumhaltige Verbindung zugegeben wird, wobei pro Atom Vanadium 1 bis 1,3 Atome Phosphor eingesetzt werden. Das während der Reaktion gebildete Wasser wird laufend und unmittelbar aus dem Reaktionsgemisch entfernt und anschliessend wird das Reaktionsgemisch aufgetrennt und der V—P—O-Komplex wird als Feststoff isoliert. Diesen Feststoff trocknet man bei Temperaturen von 90 bis 150°C, vorzugsweise 130 bis 140°C, und aktiviert diesen bei Temperaturen von 200 bis 300°C zum Katalysator.

Der erfindungsgemässe Katalysator hat eine definierte Kristallstruktur, die durch folgende Röntgenbeugungslinien (CuK$_\alpha$) charakterisiert wird:

Tabelle A

| d-Werte (Ångstrom) | Intensität | d-Werte (Ångstrom) | Intensität |
|---|---|---|---|
| 5,83 | VW | 2,65 | W |
| 5,65 | VS | 2,60 | W |
| 4,79 | W | 2,55 | VVW |
| 4,53 | VS | 2,44 | VVW |
| 4,08 | W | 2,39 | W |
| 3,68 | M | 2,25 | VVW |
| 3,29 | M | 2,22 | W |
| 3,10 | M | 2,20 | VVW |
| 2,95 | VVW | 2,12 | VW |
| 2,94 | VS | 2,10 | VVW |
| 2,78 | M | 2,04 | VW |

VS = sehr gross    W = schwach

S = gross    VW = sehr schwach

M = mittel    VVW = äusserst schwach

Die stärksten Beugungslinien stimmen dabei mit den in Tabelle A der GB-Anm. 2 118 060 angegebenen Werten überein.

Nach der Aktivierung kann der Katalysator als solcher eingesetzt werden. In einer vorzugsweisen Ausführungsform wird der Katalysator anschliessend noch reduziert. Diese Reduktionsbehandlung wird durch eine Behandlung des Katalysators mit einem gasförmigen Kohlenwasserstoff mit 2 bis 6 C-Atomen, zweckmässig Butadien, n-Butan, 1-Buten, cis-2-Buten, trans-2-Buten oder Mischungen davon und vorzugsweise n-Butan, in Abwesenheit von molekularem Sauerstoff bei Temperaturen von 300 bis 500°C ausgeführt.

Der gasförmige Kohlenwasserstoff kann auch im Gemisch mit einem Inertgas, vorzugsweise CO$_2$ oder N$_2$, eingesetzt werden.

Dieser Reduktionsprozess an sich ist in der US—PS 4 181 628 beschrieben und führt bei neuen Katalysatoren nach vorliegender Erfindung zu nochmals gesteigerter Leistungsfähigkeit und bei im Gebrauch stehenden Katalysatoren nach vorliegender Erfindung, die in ihrem Leistungsverhalten absinken, zur Wiedererlangung der ursprünglichen vollen Leistungsfähigkeit. Die Reduktion kann auch am im Reaktor eingebauten Katalysator erfolgen.

Als organisches Lösungsmittel kann ein Alkohol mit 1 bis 6 C-Atomen, zweckmässig Methanol, Ethanol, 1-Propanol, 2-Propanol, 1-Butanol, 2-Butanol, Isobutanol, iso-Amylalkohol oder Gemische davon und vorzugsweise Isobutanol eingesetzt werden.

Geeignete Phosphor enthaltende Verbindungen sind solche mit 5-wertigem Phosphor, zweckmässig P$_2$O$_5$ oder 85 Gew.%ige H$_3$PO$_4$ und vorzugsweise 100 Gew.%ige H$_3$PO$_4$.

Als vanadiumhaltige Verbindungen können anorganische und/oder organische Verbindungen des 4- und/oder 5-wertigen Vanadiums oder anorganische Verbindungen des 4-wertigen Vanadiums und vorzugsweise V$_2$O$_5$ angewendet werden.

Wesentlich für die Qualität des Katalysators ist die Form der Vanadiumverbindung. Die besten Resultate werden mit einer pulverförmigen Vanadiumverbindung erreicht, wobei der Korngrössendurchmesser gleich oder kleiner als 2 µm und vorzugsweise 1 µm oder weniger sein soll. Solche Pulverfeinheiten lassen sich beispielsweise durch Mahlen mit einer Strahlmühle erreichen.

Die Bestandteile Vanadium und Phosphor werden in einem Atom-verhältnis P/V zwischen 1:1 bis 1,3:1 eingesetzt, vorzugsweise beträgt das Verhältnis 1,05:1 bis 1,2:1.

Eine Reihe von aktivitätserhöhenden Zusätzen (Promotoren) können dem Katalysator während des Herstellungsprozesses oder vor dem Formen des fertigen Katalysators beigefügt werden. Diese lassen sich aus Li, Ti, Zr, Hf, Cr, Mo, Mn, Fe, Co, Ni, Cu, Zn, B, Si, Sn oder Bi auswählen und können allein oder in Mischung untereinander angewendet werden. Wird ein aktivitätserhöhender Zusatz angewendet, beträgt das Verhältnis Hilfsstoff zu Vanadium 0,01:1 bis 0,3:1, vorzugsweise 0,05:1 bis 0,15:1. Die obengenannten aktivitätserhöhenden Zusätze können als Oxide oder Salze, dann zweckmässig als Carbonat, Nitrat, Chlorid, Bromid, Sulfat oder Acetat angewendet werden.

Die Herstellung des Katalysators kann beispielsweise derart erfolgen, dass man in einen Reaktor, der mit einer Rührvorrichtung, einer Destillationskolonne mit Kondensator und Wasserabscheider ausgerüstet ist, das Lösungsmittel und die Phosphorverbindung gibt. Die Menge Lösungsmittel, bezogen auf 1 kg Phosphorverbindung, beträgt zweckmässig 5 bis 50 kg und vorzugsweise 10 bis 20 kg Lösungsmittel pro kg Phosphor. Die Mischung wird auf den Siedepunkt erhitzt und unter Rühren wird die Vanadiumverbindung als Feststoff oder als Suspension in einem Suspensionsmittel, das in der Regel dem genannten Lösungsmittel entspricht, langsam über einen Zeitraum von zweckmässig 0,5 bis 4 Stunden, vorzugsweise 0,8 bis 2 Stunden, in den Reaktor gegeben. Während dieses Verfahrensschrittes sinkt die Wertigkeit des Vanadiums auf weniger als 5 und das während der Rekation gebildete Wasser wird mittels des Wasserabscheiders abgetrennt. Ist die Vanadiumzugabe beendet, wird das Reaktionsgemisch noch während 1 bis 2 Stunden am Rückfluss gehalten und ebenfalls sich bildendes Wasser laufend entfernt, wobei sich für das Vanadium eine endgültige Wertigkeit von 3,9 bis 4,2 einstellt.

Die den V—P—O-Komplex enthaltende Suspension wird anschliessend gekühlt.

Das Abscheiden des sich bildenden V—P—O-Oxidgemisches kann durch Filtration und anschliessendes Trocknen erfolgen, oder das Lösungsmittel kann zur Trockene eingedampft werden, wobei in beiden Fällen das feste, getrocknete V—P—O-Oxidgemisch resultiert. Als weitere geeignete Möglichkeit kann beispielsweise durch Verdampfen oder Abdestillieren des Lösungsmittels auf eine Suspension mit einer 40—50% Feststoff-Konzentration abgezielt werden, um dann dieses schlickerartige Reaktionsgemisch durch Sprühtrocknen, gegebenenfalls mit Hilfe von Bindemitteln, weiter zu verarbeiten.

Die Trocknungstemperatur für das V—P—O-Oxidgemisch beträgt jeweils zweckmässig 90 bis 150°C, vorzugsweise 130 bis 140°C.

Die letztgenannte Methode, um das V—P—O-Oxidgemisch aus dem Reaktionsgemisch abzutrennen, ist besonders geeignet, wenn der Feststoff geformt, beispielsweise tablettiert, für die Verwendung im Festbettreaktor, werden soll. Durch das Sprühtrocknen werden Pulver mit ausgezeichneten Fliesseingenschaften erhalten, die sich leicht zu Tabletten mit sehr homogenen Eigenschaften verarbeiten lassen. Andere Formgebungssysteme, die angewendet werden können, sind beispielsweise die Extrusion oder die Tablettierung mittels eines Granuliertellers oder -trommel.

Das Formen des Katalysators kann vor oder nach der Aktivierung erfolgen, wobei die Reihenfolge zwischen Aktivieren und Formen an sich nicht kritisch ist. Die Aktivierung wird durch Erhitzen auf Temperaturen von 200 bis 300°C vorgenommen und benötigt in der Regel 2 bis 24 Stunden.

Wie oben genannt kann der Katalysator für die Verwendung von Festbettreaktoren geformt werden, oder der erfindungsgemässe Katalysator kann durch geeignete Behandlung, z.B. durch Sprühtrocknen, für die Verwendung in einem Wirbelschichtreaktor vorgesehen werden.

Der erfindungsgemässe Katalysator wird für die Oxidation von 1,3-Butadien, n-Butan, 1-Buten, cis-2-Buten, trans-2-Buten und Mischungen davon zu Maleinsäureanhydrid verwendet. Vorzugsweise wird der erfindungsgemässe Katalysator für partielle Oxidation von n-Butan zu Maleinsäureanhydrid verwendet.

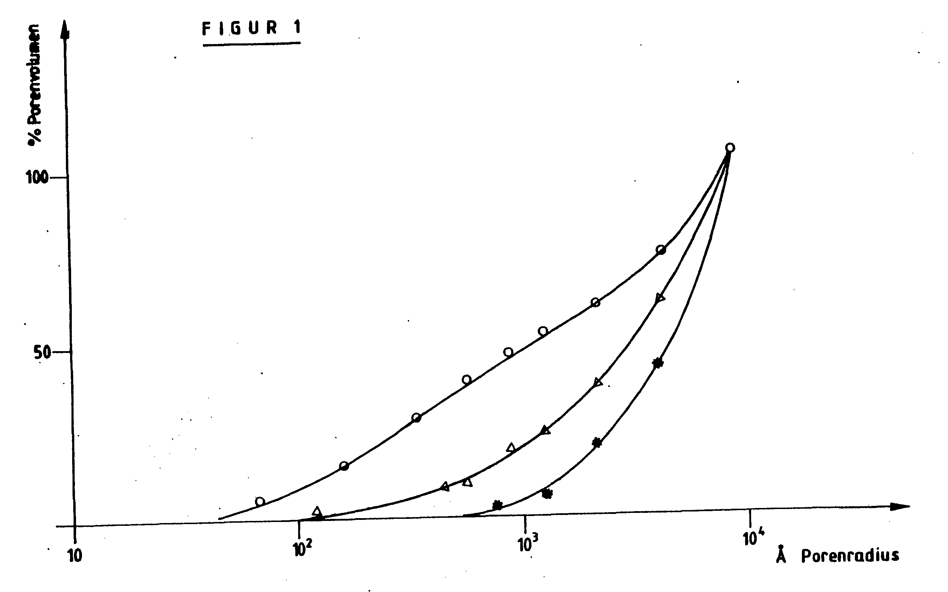

Die Abbildung 1 zeigt die Verteilung des Porenvolumens für Poren mit einem Radius von kleiner als 10'000 Å in V—P—O-Oxidgemischen, getrocknet bei jeweils 140°C.

Die Kurven bedeuten:

o V—P—O-Oxidgemisch, hergestellt nach vorliegender Erfindung (Beispiel 1);

* V—P—O-Komplex hergestellt nach US—PS 4 100 106 (Vergleichsbeispiel 1);

v V—P—O-Komplex hergestellt in Isobutanol als Lösungsmittel, alles $V_2O_5$ war von Beginn der Reaktion an im Reaktionsgemisch (Vergleichsbeispiel 2).

## Beispiele

Um die in den nachfolgenden Beispielen 1—5 hergestellten Katalysatoren zu prüfen und um die Katalysatorleistung zu ermitteln, wurden drei verschiedene Reaktoren angewendet.

Reaktor A:  Reaktordurchmesser $\phi i$ = 21 mm
Höhe des Katalysatorbettes = 900 mm
Das Reaktionsrohr ist in einen Aluminiumblock eingesetzt und durch eine elektrische Spulenheizung beheizt.

Reaktor B;  Reaktordurchmesser $\phi i$ = 21 mm
Höhe des Katalysatorbettes = 3000 mm
Das Reaktionsrohr ist in ein Salzbad eingetaucht und durch eine elektrische Spulenheizung beheizt.

Reaktor C:  Reaktordurchmesser $\phi i$ = 25 mm
Höhe des Katalysatorbettes = 3000 mm Das Reaktionsrohr ist in ein Salzbad eingetaucht und durch eine elektrische Spulenheizung beheizt.

Alle drei Reaktoren sind jeweils im Inneren mit einem axial angebrachten Thermoelement zur Temperaturmessung ausgerüstet. Die Katalysatorleistung ist jeweils durch die nachfolgend definierten Parameter bestimmt worden:

% Kohlenwasserstoff Umsatz =

$$\frac{\text{Mole Kohlenwasserstoff umgesetzt zu Produkten}}{\text{Mole Kohlenwasserstoff eingespeist}} \cdot 100$$

$$\text{Gew.\% Ausbeute} = \frac{\text{g Maleinsäureanhydrid}}{\text{g Kohlenwasserstoff eingespeist}} \cdot 100$$

$$\text{Gew.\% Selektivität} = \frac{\text{g Maleinsäure hergestellt}}{\text{g Kohlenwasserstoff umgesetzt zu Produkten}} \cdot 100$$

Beispiel 1

In einem Dreihalskolben mit 5 Liter Inhalt, ausgerüstet mit einem Thermometer, einem mechanischen Rührer, einer gepackten Glasdestillationskolonne mit Rückflusskühler und einem Dean-Stark-Wasserabscheider wurden 2 Liter Isobutanol und 404 g $H_3PO_4$ 100% eingefüllt.

Die Mischung wurde auf Rückflusstemperatur gebracht und dann eine Suspension von 326 g $V_2O_5$ in 1000 ml Isobutanol im Zeitraum von 1 Stunde zugegeben.

Während der Zugabe von $V_2O_5$ wurde die gleiche Menge an Isobutanol, die mit dem $V_2O_5$ zugeführt wurde, aus dem Reaktionsgemisch abdestilliert, wobei das während der Reaktion durch die Reduktion des $V_2O_5$ gebildete Wasser ebenfalls abdestilliert wurde.

Nach beendigter Zugabe des $V_2O_5$ wurde noch während 2 Stunden um Rückfluss gekocht, um alles während der Reaktion gebildete Wasser zu entfernen.

Der entstandene Schlicker wurde dann gekühlt und der blaue Feststoff abfiltriert und bei 140°C getrocknet.

Auf diesem beschriebenen Wege wurde ein V—P—O-Oxidgemisch als Vorläufer und Ausgangsprodukt für den eigentlichen Katalysator erhalten.

Die Verteilung der Poren des resultierenden Pulvers war derart, dass das Porenvolumen der Poren mit einem Radius von 100 bis 1'000 Å 36% des totalen Volumens der Poren mit weniger als 10'000 Å darstellen. Das wird in Abbildung 1 verdeutlicht, in der die Verteilung des Porenvolumens mit demjenigen, erhalten bei Katalysatorausgangsprodukten hergestellt nach dem Stand der Technik, verglichen wird. Siehe dazu auch die Vergleichsbeispiele 1 und 2.

Das trockene Pulver wurde zu zylinderförmigen Tabletten mit einem Durchmesser von 3 mm und einer Höhe von 3 mm mit Hilfe von Stearinsäure als Additiv gepresst. Die zylinderförmigen Tabletten wurde bei 300°C während 6 Stunden calciniert. 300 g des Katalysators wurden in den Laborreaktor A bei 300°C eingefüllt.

Der Katalysator wurde in einem Luftstrom (500 Nl/h) auf 350°C aufgeheizt, wobei die Temperatur um 20°C/h erhöht wurde. Anschliessend wurden 30 g n-Butan eingespeist. Innerhalb 2 Stunden wurde die Temperatur auf 380°C gebracht und der Reaktor während 16 Studen unter diesen Bedingungen belassen. Zu diesem Zeitpunkt erreichte der Katalysator seine beste und konstante Leistung.

<u>Tabelle 1</u> zeigt die Katalysatorleistung während des Versuches:

| Laufzeit h | Reaktortem- peratur °C | $C_4$-Umsatz % | Ausbeute Gew.% | Selektivität Gew.% |
|---|---|---|---|---|
| 16 | 380 | 90 | 110 | 122 |
| 540 | 370 | 89 | 108 | 121 |
| 960 | 375 | 91 | 109 | 120 |

EP 0 182 078 B1

Beispiel 2

Das Verfahren war an sich dasselbe wie in Beispiel 1. In einen Reaktor mit 400 Liter Kapazität aus AISI 316 rostfreiem Stahl, ausgerüstet mit Rührer, gepackter Destillationskolonne und Wasserabscheider wurden 250 l Isobutanol und 38,7 kg Phosphorsäure 100% eingefüllt.

Eine Suspension von 31,3 kg $V_2O_5$ in 100 l Isobutanol wurde kontinuierlich innerhalb einer Stunde zugegeben. Während der Zugabe des Suspension wurde die entsprechende Menge Isobutanol abdestilliert, wobei das bei der Reduktion des $V_2O_5$ gebildete Wasser gleichzeitig abgetrennt wurde.

Nach beendigter Suspensionszugabe wurde noch während 2,5 Stunden am Rückfluss gekocht und alles Ausser aus dem Gemisch entfernt. Der das V—P—O-Oxidgemisch enthaltende Schlicker wurde durch Abdestillieren des Isobutanols aufkonzentriert. Als der Feststoffgehalt des Schlickers 35 Gew.% erreicht hatte, wurde abgekühlt und sprühgetrocknet, wobei das Lösungsmittel zurückgewonnen wurde.

Das erhaltene mikrosphärische Produkt wurde einer thermischen Vorbehandlung bei 280°C während 12 Stunden in Luft unterzogen. Es wurde Stearinsäure zugegeben und das Gemisch zu 4 × 4 mm grossen Zylindern tablettiert. Diese Tabletten wurden in einen Reaktor Typ B bei 300°C eingefüllt.

Die Temperatur wurde kontinuierlich bis auf 370°C durch Luftzufuhr erhöht, bei 370°C wurde mit der Zuspeisung von n-Butan begonnen, bis der optimale Betriebsbereich erreicht war (Raum-Geschwindigkeit, "Space Velocity", 3200 $h^{-1}$, n-Butan-Konzentration 1,8 Mol%).

Nach rund 8000 Stunden Laufzeit wurde der Katalysator einem Reaktivierungsverfahren, wie in der US—PS 4 181 628 beschrieben, unterworfen. Eine n-Butan-Stickstoff-Mischung (50 Vol% n-Butan) wurde zu diesem Zweck während 12 Stunden bei 430°C in den Reaktor und über den Katalysator geleitet. Nach dieser Behandlung lautete die Leistung des Katalysators wie folgt: Salztemperatur 400°C, $C_4$-Umsatz 83%, Ausbeute Gew.% 97, Selektivität 117%.

Die Katalysatorleistung ist in Tabelle 2 gelistet.

## Tabelle 2

| Laufzeit h | Salztempera- tur °C | $C_4$-Umsatz % | Ausbeute Gew.% | Selektivität Gew.% |
|---|---|---|---|---|
| 120 | 410 | 81 | 98 | 121 |
| 720 | 407 | 82 | 99 | 121 |
| 1250 | 405 | 80 | 97 | 121 |
| 4250 | 407 | 83 | 98 | 118 |
| 7680 | 407 | 85 | 93 | 109 |

Der Katalysator kann nach längerer Betriebszeit Leistungseinbussen zeigen. Es ist leicht möglich, den Katalysator dann beispielsweise nach einem Verfahren, wie in der US—PS 4 181 628 beschrieben, zu regenerieren.

Beispiel 3

Es wurden die Vorrichtung und das Verfahren nach Beispiel 2 angewendet.

Am Ende der Reaktion wurde das V—P—O-Oxidgemisch in einer Filterpresse filtriert. Der Filterkuchen wurde bei 80°C in einem Vakuumtrockner getrocknet und nach Zugabe von Stearinsäure granuliert. Das Granulat wurde gemahlen und die Fraktion mit einer Korngrösse zwischen 50 und 100 μm wurde zu Tabletten in Ringform mit der Grösse 5 mm äusserer Durchmesser, 4 mm Höhe und 1,5 mm Dicke gepresst.

Nach der thermischen Vorbehandlung bei 300°C während 7 Stunden mit Luft wurden die Tabletten in einer Reaktor des Typs C bei 300°C eingefüllt. Die Betriebsdaten wurden auf eine Raumgeschwindigkeit von 2300 h$^{-1}$ und eine n-Butan-Konzentration von 1,8 Mol% eingestellt.

Die Katalysatorleistungen sind in Tabelle 3 festgehalten.

## Tabelle 3

| Laufzeit h | Salztempera- tur    °C | C$_4$-Umsatz % | Ausbeute Gew.% | Selektivität Gew.% |
|---|---|---|---|---|
| 130 | 398 | 85 | 102 | 120 |
| 800 | 395 | 87 | 104 | 120 |
| 1200 | 395 | 85 | 104 | 122 |
| 4000 | 397 | 86 | 103 | 120 |

### Beispiel 4

Ein V—P—O-Oxidgemisch wurde nach Beispiel 1 hergestellt, geformt und calciniert.

300 g der zylinderförmigen Stücke φ 3 mm, H 3 mm) wurden in einen Laborreaktor des Typs A bei 300°C eingefüllt. Die Reaktionsverhältnisse wurden laufend eingestellt auf:

| | |
|---|---|
| Luftstrom | 500 Nl/h |
| C$_4$-Fraktion-Zustrom | 15 g/h |
| Reaktortemperatur | 365°C. |

Die Zusammensetzung der C$_4$-Fraktion war:

| | |
|---|---|
| n-Butan | 18% |
| Isobutan | 5% |
| 1-Buten | 55% |
| cis-2-Buten | 7% |
| trans-2-Buten | 15% |

Nach 12 Stunden hatte sich der Katalysator eingependelt auf:

| | |
|---|---|
| C$_4$-Umsatz | 91% |

Gewichtsmässige Ausbeute, bezogen auf die totale Menge C$_4$ eingespeisen:

90%

### Beispiel 5

Ein Katalysator wurde hergestellt und zu Mikrokügelchen (durchschnittliche Grösse 100 µm) durch Luftsprühen, wie in Beispiel 2 beschrieben, verarbeitet. Die Mikrokügelchen wurden einer Hitzevorbehandlung bei 250°C während 8 Stunden unterworfen.

500 g dieses Katalysators wurden in einen gläsernen Flüssigbett-Laborreaktor (φi = 40 mm) gegeben. Die Temperatur wurde laufend auf 370°C durch Luftzufuhr erhöht. Bei 370°C wurde n-Butan eingespeisen und die Reaktionsbedingungen eingestellt: Luftstrom 150 Nl/h, n-Butan-Konzentration 5 Mol%. Nach 10 Stunden ist der Katalysator bezüglich seiner Leistung stabil: Reaktionstemperatur 380°C, n-Butan-Umsatz 75%, gewichtsmässige Ausbeute 92%.

### Vergleichsbeispiel 1

Ein V—P—O-Komplex wurde entsprechend dem Beispiel 1 der US—PS 4 100 106 nachgearbeitet.

1000 g V$_2$O$_5$ wurden in 8000 g einer 36%igen HCl (wässrige Lösung) suspendiert. Diese Suspension wurde sorgfältig unter Rühren aufgeheizt und bei 100°C während 2 Stunden am Rückfluss gekocht. Anschliessend wurden langsam 70 g Oxalsäure wasserfrei, gelöst in 700 ml Wasser, und schliesslich 1370 g H$_3$PO$_4$ 85%ig zugegeben. Diese Mischung wurde auf ein Volumen von 2000 ml aufkonzentriert und dann 2000 ml Wasser zu der entstandenen viskosen Lösung gegeben.

Es entstand eine blaue kristalline Fällung. Der Feststoff wurde abfiltriert und getrocknet.

Bei der Messung der Porenverteilung des Pulvers ergab sich die Kurve gemäss Abbildung 1: Die Poren mit einem Radius zwischen 100 und 1'000 Å machten lediglich 4% des totalen Porenvolumens der Poren unter 10'000 Å aus.

## Vergleichsbeispiel 2

Vergleichsweise wurde ein V—P—O-Komplex hergestellt, indem man die ganze Menge Vanadiumverbindung zu Beginn der Umsetzung vorlegte. Eine Mischung von 2 Liter Isobutanol, 404 g $H_3PO_4$ 100%ig und 326 g $V_2O_5$ wurde während 10 Stunden am Rückfluss gekocht und das Wasser, das bei der Reduktion des $V_2O_5$ und der Bildung des V—P—O-Oxidgemisches entstand, laufend abgeführt.

Der entstandene Schlicker wurde gekühlt, die Feststoffe abfiltriert und bei 130°C getrocknet.

Die Porenverteilung der Poren des entstandenen Pulvers wird in Abbildung 1 gezeigt: die Poren mit einem Radius zwischen 100 und 1'000 Å betrugen lediglich 18% des totalen Porenvolumens der Poren unter 10'000 Å. Das trockene Pulver wurde in Zylinder (ϕ 3 mm, Höhe 3 mm) geformt und bei 300°C während 6 Stunden calciniert.

300 g des Katalysators wurden in einen Reaktor des Typs A gegeben und gemäss Beispiel 1 aktiviert. Der Katalysator erreichte seine beste Leistung (88% Umsatz und 98 Gew.% Ausbeute, Reaktortemperatur 380°C) erst nach 360 Stunden Laufzeit.

## Patentansprüche

1. Katalysator für die Oxidation von Kohlenwasserstoffen zu Maleinsäureanhydrid, enthaltend ein Vanadium-Phosphor-Oxidgemisch und gegebenenfalls aktivitätserhöhende Zusätze, wobei der Katalysator pro Atom Vanadium 1 bis 1,3 Atome Phosphor enthält, dadurch gekennzeichnet, dass der Katalysator derart hergestellt wird, dass in einem organischen Lösungsmittel die phosphorhaltige Verbindung vorgelegt wird und innerhalb von 0,5 bis 4 Stunden, vorzugsweise 0,8 bis 2 Stunden, kontinuierlich die vanadiumhaltige Verbindung zugegeben wird, wobei pro Atom Vanadium 1 bis 1,3 Atome Phosphor eingesetzt werden und das während der Reaktion gebildete Wasser laufend und unmittelbar aus dem Reaktionsgemisch entfernt wird, anschliessend das Reaktionsgemisch getrennt und der V—P—O-Komplex als Feststoff isoliert und dieser bei Temperaturen von 90 bis 150°C getrocknet und bei Temperaturen von 200 bis 300°C zum Katalysator aktiviert wird.

2. Katalysator nach Patentanspruch 1, dadurch gekennzeichnet, dass wenigstens 90% der Katalysatorpartikel vor der Konfektionierung einen Durchmesser zwischen 1 und 5 µm aufweisen.

3. Verfahren zur Herstellung eines Katalysators für die Oxidation von Kohlenwasserstoffen zu Maleinsäureanhydrid, enthaltend ein Vanadium-Phosphor-Oxidgemisch und gegebenenfalls aktivitätserhöhende Zusätze, wobei der Katalysator pro Atom Vanadium 1 bis 1,3 Atome Phosphor enthält, dadurch gekennzeichnet, dass in einem organischen Lösungsmittel die phosphorhaltige Verbindung vorgelegt wird und innerhalb von 0,5 bis 4 Stunden, vorzugsweise 0,8 bis 2 Stunden, kontinuierlich die vanadiumhaltige Verbindung zugegeben wird, wobei pro Atom Vanadium 1 bis 1,3 Atome Phosphor eingesetzt werden und das während der Reaktion gebildete Wasser laufend und unmittelbar aus dem Reaktionsgemisch entfernt wird, anschliessend das Reaktionsgemisch getrennt und der V—P—O-Komplex als Feststoff isoliert und dieser bei Temperaturen von 90 bis 150°C getrocknet und bei Temperaturen von 200 bis 300°C zum Katalysator aktiviert wird.

4. Verfahren nach Patentanspruch 3, dadurch gekennzeichnet, dass als organisches Lösungsmittel ein Alkohol mit 1 bis 6 C-Atomen, zweckmässig Methanol, Ethanol, 1-Propanol, 2-Propanol, 1-Butanol, 2-Butanol, Isobutanol, iso-Amylalkohol oder Gemische davon und vorzugsweise Isobutanol angewendet wird.

5. Verfahren nach Patentansprüchen 3 und 4, dadurch gekennzeichnet, dass als phosphorhaltige Verbindungen solche, enthaltend 5-wertigen Phosphor, zweckmässig $P_2O_5$ oder 85 Gew.%ige $H_3PO_4$ und vorzugsweise 100 Gew.%ige $H_3PO_4$, angewendet werden.

6. Verfahren nach Patentansprüchen 3 bis 5, dadurch gekennzeichnet, dass als vanadiumhaltige Verbindungen anorganische Verbindungen des 5-wertigen Vanadiums oder anorganische Verbindungen des 4-wertigen Vanadiums und vorzugsweise $V_2O_5$ angewendet wird.

7. Verfahren nach Patentansprüchen 3 bis 6, dadurch gekennzeichnet, dass man die vanadiumhaltige Verbindung in einer Korngrösse von gleich oder kleiner als 2 µm und vorzugsweise von 1 µm oder weniger anwendet.

8. Verfahren nach Patentansprüchen 3 bis 7, dadurch gekennzeichnet, dass pro Atom Vanadium 1,05 bis 1,2 Atome Phosphor eingesetzt werden.

9. Verfahren nach Patentansprüchen 3 bis 8, dadurch gekennzeichnet, dass im Verlauf des Herstellungsverfahrens ein ein aktivitätserhöhender Zusatz aus der Reihe von Li, Ti, Zr, Hf, Cr, Mo, Mn, Fe, Co, Ni, Cu, Zn, B, Si, Sn oder Bi, allein oder in einem Gemisch untereinander, im Verhältnis aktivitätserhöhender Zusatz zu Vanadium 0,01:1 bis 0,3:1, vorzugsweise 0,05:1 bis 0,15:1, zugegeben wird.

10. Verfahren nach Patentansprüchen 3 bis 9, dadurch gekennzeichnet, dass der aktivitätserhöhender Zusatz als Oxid oder Salz, zweckmässig als Carbonat, Nitrat, Chlorid, Bromid, Sulfat oder Acetat zugegeben wird.

11. Verfahren nach Patentansprüchen 3 bis 10, dadurch gekennzeichnet, dass man den Feststoff bei 90 bis 150°C, vorzugsweise bei 130 bis 140°C, trocknet.

# EP 0 182 078 B1

12. Verfahren nach Patentansprüchen 3 bis 11, dadurch gekennzeichnet, dass der Katalysator nach dem Aktivieren oder zum Reaktivieren durch Behandeln mit einem gasförmigen Kohlenwasserstoff mit 2 bis 6 C-Atomen, zweckmässig 1,3-Butadien, n-Butan, 1-Buten, cis-2-Buten, trans-2-Buten oder Mischungen davon und vorzugsweise n-Butan, in Abwesenheit von molekularem Sauerstoff bei Temperaturen von 300 bis 500°C reduziert wird.

13. Verfahren nach Patentanspruch 12, dadurch gekennzeichnet, dass der gasförmige Kohlenwasserstoff im Gemisch mit einem Inertgas, vorzugsweise $CO_2$ oder $N_2$, eingesetzt wird.

14. Verwendung des Katalysators nach Patentanspruch 1 für die partielle Oxidation von 1,3-Butadien, n-Butan, 1-Buten, cis-2-Buten, trans-2-Buten und Mischungen davon, vorzugsweise von n-Butan, zu Maleinsäureanhydrid.

## Revendications

1. Catalyseur pour l'oxydation d'hydrocarbures en anhydride maléique, contenant un mélange vanadium-phosphore-oxyde et le cas échéant des additifs augmentant l'activité, le catalyseur contenant par atome de vanadium 1 à 1,3 atome de phosphore, caractérisé en ce que le catalyseur est préparé en introduisant dans un solvant organique le composé phosphoré et en ce que en un laps de temps de 0,5 à 4 heures, de préférence de 0,8 à 2 heures, le composé à base de vanadium est ajouté en continu, moyennant quoi par atome de vanadium on met en oeuvre 1 à 1,3 atome de phosphore et l'eau formée pendant la réaction est éliminée continuellement et directement du mélange réactionnel, après quoi le mélange réactionnel est séparé et le complexe V—P—O est isolé en tant que matière solide et celle-ci est séchée à des températures de 90 à 150°C et activée en catalyseur à des températures de 200 à 300°C.

2. Catalyseur selon la revendication 1, caractérisé en ce qu'au moins 90% des particules de catalyseur présentent un diamètre entre 1 et 5 μ avant confection.

3. Procédé pour la préparation d'un catalyseur pour l'oxydation d'hydrocarbures en anhydride maléique contenant un mélange vanadium-phosphore-oxyde et le cas échéant des additifs augmentant l'activité, le catalyseur contenant par atome de vanadium 1 à 1,3 atome de phosphore, caractérisé en ce qu l'on introduit le composé phosphoré dans un solvant organique et en ce que l'on ajoute en un laps de temps de 0,5 à 4 heures, de préférence de 0,8 à 2 heures, de façon continue, le composé à base de vanadium, moyennant quoi par atome de vanadium on met en oeuvre 1 à 1,3 atome de phosphore et l'eau formée pendant la réaction est éliminée continuellement et directement du mélange réactionnel, après quoi le mélange réactionnel est séparé et le complexe V—P—O est isolé en tant que matière solide et celle-ci est séchée à des températures de 90 à 150°C et activée en catalyseur à des températures de 200 à 300°C.

4. Procédé selon la revendication 3, caractérisé en ce qu'en tant que solvant organique on utilise un alcool ayant 1 à 6 atomes de C, de façon judicieuse le méthanol, l'éthanol, le 1-propanol, le 2-propanol, le 1-butanol, le 2-butanol, l'isobutanol, l'isoamylalcool ou des mélanges de ceux-ci et de préférence l'isobutanol.

5. Procédé selon les revendications 3 et 4, caractérisé en ce qu'en tant que composés phosphorés on utilise ceux contenant du phosphore pentavalent, de façon judicieuse $P_2O_5$ ou 85% en poids d'$H_3PO_4$ et de préférence 100% en poids d'$H_3PO_4$.

6. Procédé selon les revendications 3 à 5, caractérisé en ce qu'en tant que composés à base de vanadium on utilise des composés inorganiques du vanadium pentavalent ou des composés inorganiques du vanadium tétravalent et de préférence $V_2O_5$.

7. Procédé selon les revendications 3 à 6, caractérisé en ce que l'on utilise le composé à base de vanadium avec une granulométrie égale ou inférieure à 2 μ et de préférence de 1 μ ou moins.

8. Procédé selon les revendications 3 à 7, caractérisé en ce que par atome de vanadium on met en oeuvre 1,05 jusqu'à 1,2 atomes de phosphore.

9. Procédé selon les revendications 3 à 8, caractérisé en ce qu'au cours du procédé de préparation on ajoute un additif augmentant l'activité choisi dans la série Li, Ti, Zr, Hf, Cr, Mo, Mn, Fe, Co, Ni, Cu, Zn, B, Si, Sn ou Bi, seul ou en combinaison entre eux, dans un rapport entre l'additif augmentant l'activité et le vanadium de 0,01:1 jusqu'à 0,3:1, de préférence de 0,05:1 jusqu'à 0,15:1.

10. Procédé selon les revendications 3 à 9, caractérisé en ce que l'additif augmentant l'activité est ajouté en tant qu'oxyde de sel, de façon judicieuse en tant que carbonate, nitrate, chlorure, bromure, sulfate ou acétate.

11. Procédé selon les revendications 3 à 10, caractérisé en ce que l'on sèche la matière solide à une température de 90°C à 150°C, de préférence de 130°C à 140°C.

12. Procédé selon les revendications 3 à 11, caractérisé en ce que le catalyseur est réduit après l'activation ou pour la réactivation par traitement avec un hydrocarbure gazeux ayant 2 à 6 atomes de C, de façon judicieuse le 1,3-butadiène, le n-butane, le 1-butène, le cis-2-butène, le trans-2-butène ou des mélanges de ceux-ci et de préférence le n-butane, en absence d'oxygène moléculaire à des températures de 300 à 500°C.

13. Procédé selon la revendication 12, caractérisé en ce que l'hydrocarbure gazeux est mis en oeuvre en mélange avec un gaz inerte, de préférence $CO_2$ ou $N_2$.

14. Utilisation du catalyseur selon la revendication 1 pour l'oxydation partielle de 1,3-butadiène, n-

# EP 0 182 078 B1

butane, 1-butène, cis-2-butène, trans-2-butène et des mélanges de ceux-ci, de préférence de n-butane, en anhydride maléique.

**Claims**

1. Catalyst for the oxidation of hydrocarbons to maleic anhydride, containing a vanadium-phosphorus oxide mixture and optionally activating additives, the catalyst containing 1 to 1.3 atoms of phosphorus per atom of vanadium, characterized in that the catalyst is produced by charging the phosphorus-containing compound in an organic solvent and adding the vanadium-containing compound continuously over 0.5 to 4 hours, preferably 0.8 to 2 hours, 1 to 1.3 atoms of phosphorus being used per atom of vanadium, and the water formed during the reaction is continuously and immediately removed from the reaction mixture, the reaction mixture is afterwards separated and the V—P—O complex isolated as solid and the latter dried at temperatures of 90 to 150°C and activated to the catalyst at temperatures of 200 to 300°C.

2. Catalyst as claimed in claim 1, characterized in that at least 90% of the catalyst particles before the forming have a diameter between 1 and 5 µm.

3. Process for the production of a catalyst for the oxidation of hydrocarbons to maleic anhydride, containing a vanadium-phosphorus oxide mixture and optionally activating additives, the catalyst containing 1 to 1.3 atoms of phosphorus per atom of vanadium, characterized in that the phosphorus-containing compound is charged in an organic solvent and the vanadium-containing compound is added continuously over 0.5 to 4 hours, preferably 0.8 to 2 hours, 1 to 1.3 atoms of phosphorus being used per atom of vanadium, and the water formed during the reaction is continuously and immediately removed from the reaction mixture, the reaction mixture is afterwards separated and the V—P—O complex isolated as solid and the latter dried at temperatures of 90 to 150°C and activated to the catalyst at temperatures of 200 to 300°C.

4. Process as claimed in claim 3, characterized in that an alcohol with 1 to 6 carbon atoms, suitably methanol, ethanol, 1-propanol, 2-propanol, 1-butanol, 2-butanol, isobutanol, isoamyl alcohol or mixtures thereof, and preferably isobutanol, is used as organic solvent.

5. Process as claimed in claims 3 and 4, characterized in that phosphorus-containing compounds containing 5-valent phosphorus, suitably $P_2O_5$ or 85 wt. % $H_3PO_4$ and preferably 100 wt. % $H_3PO_4$, are used as phosphorus-containing compounds.

6. Process as claimed in claims 3 to 5, characterized in that inorganic compounds of 5-valent vanadium or inorganic compounds of 4-valent vanadium, and preferably $V_2O_5$, are used as vanadium-containing compounds.

7. Process as claimed in claims 3 to 6, characterized in that the vanadium-containing compound is used in a particle size of 2 µm or less and preferably of 1 µm or less.

8. Process as claimed in claims 3 to 7, characterized in that 1.05 to 1.2 atoms of phosphorus are used per atom of vanadium.

9. Process as claimed in claims 3 to 8, characterized in that during the course of the production process an activating additive from the set Li, Ti, Zr, Hf, Cr, Mo, Mn, Fe, Co, Ni, Cu, Zn, B, Si, Sn or Bi is added, alone or in a mixture with one another, in the ratio of activating additive to vanadium of 0.01:1 to 0.3:1, preferably 0.05:1 to 0.15:1.

10. Process as claimed in claims 3 to 9, characterized in that the activating additive is added as oxide or salt, suitably as carbonate, nitrate, chloride, bromide, sulfate or acetate.

11. Process as claimed in claims 3 to 10, characterized in that the solid is dried at 90 to 150°C, preferably at 130 to 140°C.

12. Process as claimed in claims 3 to 11, characterized in that the catalyst after activation or for reactivation is reduced by treatment with a gaseous hydrocarbon with 2 to 6 C atoms, suitably 1,3-butadiene, n-butane, 1-butene, cis-2-butene, trans-2-butene or mixtures thereof, and preferably n-butane, in the absence of molecular oxygen at temperatures of 300 to 500°C.

13. Process as claimed in claim 12, characterized in that the gaseous hydrocarbon is used in a mixture with an inert gas, preferably $CO_2$ or $N_2$.

14. Use of the catalyst as claimed in claim 1 for the partial oxidation of 1,3-butadiene, n-butane, 1-butene, cis-2-butene, trans-2-butene and mixtures thereof, preferably of n-butane, to maleic anhydride.

11

FIGUR 1